(19) Europäisches Patentamt
European Patent Office
Office européen des brevets

(11) **EP 2 073 928 B1**

(12) **EUROPÄISCHE PATENTSCHRIFT**

(45) Veröffentlichungstag und Bekanntmachung des Hinweises auf die Patenterteilung:
**12.10.2016 Patentblatt 2016/41**

(21) Anmeldenummer: **07821427.7**

(22) Anmeldetag: **17.10.2007**

(51) Int Cl.:
*B01J 23/28* [(2006.01)]   *C07C 45/33* [(2006.01)]
*B01J 27/057* [(2006.01)]   *B01J 27/19* [(2006.01)]
*B01J 23/24* [(2006.01)]   *B01J 23/652* [(2006.01)]
*B01J 23/887* [(2006.01)]

(86) Internationale Anmeldenummer:
**PCT/EP2007/061062**

(87) Internationale Veröffentlichungsnummer:
**WO 2008/046843 (24.04.2008 Gazette 2008/17)**

(54) **verwendung von MISCHOXIDKATALYSATOREN FÜR DIE KATALYTISCHE GASPHASENOXIDATION von alkanen**

use of MIXED OXIDE CATALYSTS FOR CATALYTIC GAS PHASE OXIDATION of alkanes

Utilisation de CATALYSEURS D'OXYDES MIXTES POUR L'OXYDATION CATALYTIQUE EN PHASE GAZEUSE d'alcanes

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **20.10.2006   CN 200610131008**

(43) Veröffentlichungstag der Anmeldung:
**01.07.2009 Patentblatt 2009/27**

(73) Patentinhaber: **Evonik Degussa GmbH**
**45128 Essen (DE)**

(72) Erfinder:
• **FISCHER, Achim**
**63739 Aschaffenburg (DE)**
• **LU, Weimin**
**Hangzhou Zhejiang 310028 (CN)**
• **WECKBECKER, Christoph**
**63584 Gründau-Lieblos (DE)**
• **HUTHMACHER, Klaus**
**63571 Gelnhausen (DE)**

(56) Entgegenhaltungen:
EP-A- 1 075 871   WO-A-2007/042369
WO-A1-99/20590   FR-A- 1 447 982
GB-A- 1 086 523   GB-A- 1 286 083
US-A- 3 776 952   US-A1- 2003 236 163

**Beschreibung**

Die Erfindung betrifft die Verwendung von Mischoxidkatalysatoren für die katalytische Gasphasenoxidation von Alkanen.

Insbesondere kann der Katalysator zur Umsetzung von Propan zu Acrolein und Acrylsäure oder Isobutan zu Methacrolein und Methacrylsäure eingesetzt werden, wobei Acrolein oder Methacrolein zumindest als Hauptprodukt gebildet werden. Die Umsetzung des Alkans an heterogenen Katalysatoren mit einem Sauerstoff enthaltenden Gas führt neben dem erwünschten Produkt Acrolein und Acrylsäure zu einer Reihe von Nebenprodukten: beispielsweise zur Bildung von $CO_2$ und CO.

Es ist bekannt, dass durch die Art der chemischen Zusammensetzung des Mischoxides (Phasenbildung und Bildung von Reaktionszentren) wie auch durch die Art des physikalischen Aufbaus (z. Bsp. Porosität, Oberflächengröße, Form des Katalysators) und die Art der Wärmeabfuhr die Fähigkeit zur Produktbildung (Selektivität) und die Produktivität (Raum - Zeitausbeute) stark beeinflusst werden können. Im Fall der Alkan-Oxidation werden als Katalysator in der Regel Mischoxide eingesetzt, die in ihrem chemischen und physikalischen Aufbau eine komplexe Struktur aufweisen.

STAND DER TECHNIK

Die Verwendung von Mischoxidkatalysatoren auf Basis von Molybdän in der Gasphasenoxidation von Olefinen zu ungesättigten Aldehyden und gegebenenfalls den korrespondierenden ungesättigten Carbonsäuren ist aus der GB 3,776,952 A, der FR 1,447,982 A, der GB 1,086,523 A, der GB 1,286,083 A und der WO 2007/042369 A1 bekannt.

Aus der EP 1 075 871 A1 ist ferner die Verwendung von Mischoxidkatalysatoren auf Basis von Molybdän in der Gasphasenoxidation von Olefinen zu ungesättigten Nitrilen bekannt.

Die Umsetzung von Propan mit Sauerstoff oder Luft über den bekannten Katalysatoren Mo-V-Te führt im allgemeinen zur Bildung von Acrylsäure. Acrolein wird gar nicht oder nur in Spuren gebildet. Die Publikationen zur Herstellung von Acrylsäure sind zahlreich und Gegenstand vieler wissenschaftlicher Diskussionen.

Die WO 2004/105938 A2 betrifft die Herstellung von Acrylsäure aus Propan unter Verwendung von Katalysatoren der allgemeinen Formel $Te_aMo_1V_bNb_cO_x$ (I) oder $Sb_a'Mo_1V_bO_y$ (I') dabei wird Acrolein mit Selektivitäten von 0 bis 0,3 % gebildet.

In der WO 2006/058998 A2 wird die Herstellung eines Katalysators mit Tantal der allgemeinen Formel $Mo-V_aXbTa_cSi_dO_x$) beschrieben. Das Katalysatorsystem wird ebenfalls zur Herstellung von Acrylsäure eingesetzt. Acrolein wird mit Selektivitäten kleiner 1% als Nebenprodukt gebildet.

Aus der US 2005/0065370 A1 ist ein Verfahren zur Herstellung von Meth(acrylsäure) durch die Umsetzung von gesättigten Kohlenwasserstoffe über einem MischoxidKatalysator, der die Elemente Mo and V, und wenigstens Te und Sb, sowie eines der Elemente Nb, Ta, W, Ce und Ti enthält, indem das Katalysatorbett durch eine weiteres Katalysatorbett unterbrochen ist, das aus einem Mischoxidkatalysator mit den Elementen Mo, Bi and Fe besteht. Dabei werden in den Beispielen nur Aussagen über die Acrylsäureselektivität gemacht.

In Xiamen Daxue Xuebao, Ziran Kexueban (2004), 43(2), 203-207 wird die Umsetzung von Propan zu Acrolein über Mo-V-Te Katalysatoren beschrieben, dabei werden Propan Umsätze um 20% und Acrolein Selektivitäten von bis zu 30% erhalten. In Cuihua Xuebao (2002), 23(3), 281-284 werden Umsätze von Propan zu Acrolein über $Ag_{0.3}MoP_{0.6}O$ und $Ce_{0.1}Ag_{0.3}MoP_{0.6}O_y$ beschrieben.

Gemäß US 2006/0004226 A1 werden Acrolein und Acrylsäure gebildet, indem Propan über zwei Reaktionszonen (Katalysatoren) umgesetzt wird. Hierbei wird in der ersten Stufe ein Gemisch aus Propan und Propen gebildet, nach der zweiten Stufe enthält das Kondensat u.a. 98,48 Gew.-% an Acrylsäure und 0,002 Gew-% Acrolein.

Aus der US 2003/0236163 A1 und der WO 99/20590 A1 ist die Oxidation von Alkanen in Gegenwart von Mischoxidkatalysatoren auf Basis von Molybdän bekannt.

Aufgabe und Abgrenzung

Die vorliegende Erfindung hat zur Aufgabe ein Verfahren zur Herstellung von Aldehyden bereitzustellen, bei dem insbesondere Acrolein oder auch Gemische aus Acrolein und Acrylsäure in guten Ausbeuten aus Propan durch Oxidation mit Sauerstoff gebildet werden. Die Oxidation wird gegebenenfalls in Gegenwart von inerten Gasen, Wasserdampf oder Abgasen bei erhöhten Temperaturen und in Gegenwart eines heterogenen Mischoxidkatalysators durchgeführt.

Die Umsetzung des Alkans zu den Oxidationsprodukten Aldehyd und Säure erfolgt bei erhöhter Temperatur und einem Verhältnis zwischen Alkan, Sauerstoff, Inertgas(en) und Wasser von im allgemeinen 1 : 0,5 - 5 : 0 - 10: 0 - 18. In einer bevorzugten Ausführungsform liegt der Wasseranteil bei 0.

Als Inertgase lassen sich alle gasförmigen Verbindungen einsetzen, die sich unter den beschriebenen Oxidationsbedingungen inert verhalten. Beispielsweise kann es sich hierbei um Stickstoff, Helium, oder Gemische aus diesen handeln. Ebenfalls möglich ist es, das "Rückgas" aus dem Reaktor wieder einzuspeisen.

Gegenstand der Erfindung ist daher die Verwendung von Mischoxidkatalysatoren der allgemeinen Formel

$$(Mo_1 \, Cr_{0,005-0,1} \, P_{0,01-0,1} \, Te_{0,01-0,8} \, G_g)O_x \qquad (I),$$

zur katalytischen Gasphasenoxidation von Alkanen,
in der bedeuten:

G: mindestens eines der Elemente umfassend Si, Al, Ti, Zr,
und

$$g = 0 - 800,$$

x = Zahl, die von der Wertigkeit und Häufigkeit der von Sauerstoff verschiedenen Elemente bestimmt wird.

Die Buchstaben a bis x repräsentieren die Atomverhältnisse der entsprechenden Elemente im Verhältnis zu Mo. Katalysatoren mit der allgemeinen Formel I sind besonders geeignete katalytisch aktive Festkörper z. B. zur Umsetzung von Propan zu Acrolein und Acrylsäure und werden mit einem Verfahren hergestellt, das ebenso offenbart ist. Besonders vorteilhaft führt man die Umsetzung in Reaktoren durch, die es erlauben, den Katalysator als Festbett oder Wirbelbett einzusetzen. Es ist aber ebenfalls möglich, den Katalysator auf die Wand des Reaktionsraumes aufzubringen. An dieser Stelle sei bemerkt, dass Katalysatoren der allgemeinen Formel I auch zur Umsetzung von iso-Butan zu Methacrolein und Methacrylsäure benutzt werden können.

Nach aus dem Stand der Technik bekannten Verfahren wird ausgehend von geeigneten Quellen der Komponenten der Multimetalloxidmasse ein möglichst inniges, vorzugsweise feinteiliges, Trockengemisch erzeugt und dieses bei Temperaturen von > 150 bis 700°C, bevorzugt 400 bis 700°C oder insbesondere 450 bis 600°C thermisch behandelt. Die thermische Behandlung kann prinzipiell sowohl unter oxidierender als auch unter inerter Atmosphäre und gegebenenfalls in Anwesenheit von Wasserdampf erfolgen. Als oxidierende Atmosphäre kommt z. B. Luft, mit molekularem Sauerstoff angereicherte Luft oder an Sauerstoff entreicherte Luft in Betracht. Vorzugsweise wird die thermische Behandlung jedoch unter inerter Atmosphäre, d. h., z. B. unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Insgesamt kann die thermische Behandlung von 0,25 h bis zu 24 h oder mehr in Anspruch nehmen und in mehreren Schritten erfolgen. Bevorzugt sind 0,25 bis 10 h.

Bevorzugt erfolgt die thermische Behandlung des trockenen Gemischs unter inerter Atmosphäre bei einer Temperatur von > 150 bis 400°C bzw. 250 bis 450°C
(=Vorzersetzungsschritt). Im Anschluß daran wird die thermische Behandlung zweckmäßig unter Inertgas bei Temperaturen von > 450 bis 700°C, insbesondere 450 bis 600°C fortgesetzt.

Sollte eine Formgebung erforderlich sein, dann ist es vorteilhaft, die thermische Behandlung im Temperaturbereich von 420 bis 490°C zu unterbrechen, die Formgebung durchzuführen, um dann im Temperaturbereich von 490 bis 700°C insbesondere bis 600°C, die thermische Behandlung fortzuführen. Es ist jedoch auch möglich, das fertig kalzinierte Pulver in Form zu bringen.

Das innige Vermischen der Ausgangsverbindungen kann in trockener oder in nasser Form erfolgen.

Erfolgt die Herstellung in nasser Form, werden die Ausgangsverbindungen in Form von wässrigen Lösungen und/oder Suspensionen miteinander vermischt. Anschließend wird die Mischung im allgemeinen bei 60°C bis < 150° C, insbesondere bis 130°C getrocknet und nach der Trocknung thermisch behandelt.

Als Quellen für die für die Bestandteile des Mischoxidkatalysators gemäß Formel (I) kommen im Rahmen der Durchführung der vorstehend beschriebenen Herstellweise alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/oder Hydroxide der elementaren Konstituenten mitverwendet oder ausschließlich verwendet werden.

Die Komponenten werden idealerweise in Form ihrer Verbindungen, ausgewählt aus der Gruppe Ammoniumverbindungen, Oxalate, Hydroxide, Carbonate-, Phosphate-, Acetate, Carbonyle und/oder Nitrate, einzeln oder gemeinsam gelöst und miteinander vermischt. Besonders bevorzugt sind, Carbonate, Nitrate und Phosphate oder Mischungen aus diesen. Ebenfalls eingesetzt werden können Säuren der Salze, beispielsweise Salpetersäure, Phosphorsäure oder Kohlensäure oder Suspensionen der entsprechenden Metalloxide.

Abhängig von der Art der Metallsalze, die in die Fällung eingesetzt werden, kann es erforderlich sein, Salze und Säuren oder Mischungen aus diesen dem Fällungsgemisch zuzugeben. Idealerweise verwendet man hierbei Ammoniak oder Ammonium-Salze, beispielsweise Ammoniumcarbonat, Ammoniumheptamolybdat oder Metallnitrate, beispielsweise Cobaltnitrat; man kann ebenfalls die entsprechenden Säuren, beispielsweise Salpetersäure in den zur Einstellung des Ionenverhältnisses notwendigen Mengen einsetzen. Der pH-Wert während der Fällung liegt üblicherweise bei < 8, insbesondere < 7.

Ebenfalls von Bedeutung ist die Temperatur der Fälllösung. So kann es sein, dass bei zu hoher Temperatur die spätere Aktivität des Katalysators deutlich reduziert wird. Die Fällung kann prinzipiell bei Temperaturen von 25 bis 90°C durchgeführt werden.

Das Kopräzipitat kann in einer Stufe gefällt werden. Besonders bevorzugt ist es, die Fällung mehrstufig durch schrittweise Zugabe der Einzelkomponenten oder Mischungen aus diesen durchzuführen. Die Anzahl der Fällstufen ist prinzipiell nicht limitiert. Bevorzugt sind aber ein bis drei Fällstufen.

Die erhaltene Suspension kann direkt weiterverarbeitet werden oder man lässt sie vorteilhaft für > 0 bis 24 Stunden reifen. Bevorzugt sind > 0 bis 12 Stunden, besonders bevorzugt sind 0 bis 6 Stunden. Es ist selbstverständlich, dass die Fällsuspension vor der Weiterverarbeitung beispielsweise durch Rühren homogenisiert wird.

Nach der Reifung kann die Flüssigkeit der Suspension durch Verdampfung, Zentrifugation oder Filtration entfernt werden. Die Flüssigkeit zu verdampfen und gleichzeitig den Feststoff zu trocknen ist ebenfalls möglich und kann beispielsweise durch Sprühtrocknung erfolgen. Die Flüssigkeit sollte bei einer Temperatur von 80 bis 130°C verdampft werden. Die Trocknung des Feststoffes kann mit Luft, sauerstoffhaltigen Inertgasen oder Inertgasen, beispielsweise Stickstoff erfolgen. Führt man die Trocknung in einem Ofen durch, so sollte die Temperatur zwischen 100 und 150°C liegen. In einem Sprühtrockner sollte die Anfangstemperatur der Trockenmediums von 200 bis 500°C und eine Temperatur bei Abscheidung des getrockneten Pulvers von 80 bis 200 °C vorsehen. Das erhaltene Korn sollte bevorzugt eine Korngrößenverteilung von 15 bis 160 $\mu$m mit einem mittleren Korndurchmesser zwischen 15 und 80 $\mu$m besitzen.

Das getrocknete Pulver kann prinzipiell in den unterschiedlichsten Ofentypen wie z. Bsp. in einem Umluftofen, Drehrohr, Hordenofen, Schachtofen oder Bandofen kalziniert werden. Die Regelgüte bzw. die Güte der Temperaturerfassung des Ofens sollte möglichst hoch sein. Die Verweildauer des Pulvers im Ofen sollte je nach Ofentyp zwischen 0,25 und 10 h betragen.

Ebenfalls ist es möglich, die Kalzinierung und die dabei eintretende thermische Zersetzung der Salze in einer oder mehreren Stufen durchzuführen. Dabei können Temperaturen von 200 bis 650 °C, insbesondere 300° bis 650° benutzt werden. Die thermische Zersetzung kann unter Zusatz von Inertgas, aus Gemischen von Sauerstoff mit einem Inertgas durchgeführt werden.

Als Inertgas einsetzbar sind z. B. Stickstoff, Helium, Wasserdampf oder Gemische dieser Gase.

Nach der thermischen Behandlung kann die erhaltene Katalysatormasse zweckmäßigerweise zerkleinert und gegebenenfalls klassiert werden.

Das so erhaltene Pulver ist als Katalysator geeignet. Für die industrielle Anwendung ist es besonders zweckmäßig, nach Zugabe von handelsüblichen Form- und Bindemittel, das Pulver zu verformen. Dies kann durch Tablettierung, Extrusion oder durch Beschichtung eines Trägers geschehen. Die geometrische Form des Trägers ist hierbei nicht limitierend. Er richtet sich vielmehr nach den Vorgaben des Reaktors (z.B. Rohrdurchmesser, Länge der Katalysatorschüttung). Beispielsweise kann der Träger eine Pyramide, ein Zylinder, ein Sattel, eine Kugel, ein Ring oder ein Vieleck sein, er kann aber auch eine Wand des Reaktors sein, in dem die Umsetzung der Reaktanten stattfindet.

Als Material für die Trägerkörper kommen erfindungsgemäß insbesondere Aluminiumoxid, Titanoxid, Silicumdioxid, Silicate wie Ton, Kaolin, Bims, Aluminiumsilicat und Magnesiumsilicat, Siliciumcarbid und Zirkonoxid in Betracht.

Die Oberfläche des Trägerkörpers kann sowohl glatt als auch rau sein. Mit Vorteil ist die Oberfläche des Trägerkörpers rau.

Die Dicke der auf den Schalenkatalysatoren befindlichen aktiven Oxidmassenschale liegt überlicherweise bei 10 bis 1000 $\mu$m. Sie kann aber auch 50 bis 700$\mu$, 100 bis 600$\mu$, oder 150 bis 400$\mu$ betragen. Mögliche Schalendicken sind auch 10 bis 500$\mu$m, 100 bis 400$\mu$, oder 150 bis 300$\mu$m.

Als Bindemittel können diverse Öle, Polyole wie beispielsweise Glycerin und Polyvinylalkohole, Cellulosen, Sacharide, Acrylate sowie Alkyl-Derivate, Mischungen oder Kondensate aus denselben eingesetzt werden. Bei einer Formgebung des Katalysatorpulvers sollte der Katalysator bevorzugt im Temperaturbereich von 490 bis 650°C thermisch nachbehandelt werden, so dass sich die Aktivmasse für den Einsatz in industriellen Reaktoren verfestigt.

Die erfindungsgemäße Reaktion zur Herstellung von Acrolein oder gebenenfalls Gemischen von Acrolein und Acrylsäure wird im allgemeinen bei Temperaturen von 350 - 500 °C und einem Druck von 1,0 - 2,2 bara durchgeführt. Die Umsetzung der Reaktionspartner Alkan zu den Oxidationsprodukten Aldehyd und gegebenenfalls Säure erfolgt bei höheren Temperaturen und einem Verhältnis zwischen Alkan, Sauerstoff, Inertgas(en) und Wasser von bevorzugt 1 : 0,5 - 5 : 0 - 10 : 0 - 15, bei einer Belastung mit 2 - 20 mol Alkan oder eines Gemisches aus Alkan /l Katalysatorschüttung/h.

Anstelle von Inertgas kann das Abgas aus der Reaktion verwendet werden, aus dem die kondensierbaren Bestandteile abgetrennt wurden. Besonders gute Ergebnisse werden beim Einsatz von Rohrbündel-, Platten- (z. Bsp. EP, 0 995 491; EP 1 147 807) oder Wandreaktoren (z.B. Redlingshoefer H., Fischer A., et al., Ind. Eng. Chem. Res. 2003, 42, 5482-5488; EP 1 234 612) erhalten, bei denen der Katalysator auf die Wand aufgebracht ist.

Der innere Durchmesser der Reaktionsrohre bzw. der Abstand der Platten sollte 18 bis 28 mm, bevorzugt 20 bis 26 mm betragen, die Wandstärke des eisenhaltigen Stahls sollte zwischen 1 und 3,5 mm liegen. Eine typische Reaktorlänge beträgt 3,00 bis 4,00 m. Der Katalysator wird bevorzugt auf der Reaktorlänge einheitlich ohne Verdünnung mit Verdünnungsformkörpern eingesetzt, natürlich kann es die Anwendung erforderlich machen, beispielsweise mit inerten Formkörpern zu verdünnen.

Die erfindungsgemäß verwendeten Katalysatoren besitzen auch bei hoher spezifischer Belastung eine verbesserte Aktivität und Selektivität für die Herstellung von Acrolein.

Die Erfindung wird nachfolgend anhand von Ausführungsbeispielen erläutert. Dabei ist definiert die Ausbeute (%) des Produktes als

$$(mol/h \ Produkt \ gebildet) \ / \ (mol/h \ Reaktant \ zugeführt) \ * \ 100$$

der Umsatz des Alkans (%) als

$$[1 - (mol/h \ aus \ dem \ Reaktionsrohr \ austretendes \ Alkan)/(mol/h \ in \ das \ Reaktionsrohr \ eintretendes \ Alkan)] \ * \ 100$$

die Selektivität (%) als

$$(Ausbeute \ des \ Produktes \ / \ Umsatz) \ * \ 100$$

Die dargestellte Erfindung wird, um das Verständnis zu verbessern, durch die nachstehenden Beispiele beschrieben, sie ist jedoch nicht auf diese Beispiele beschränkt.

BEISPIELE

Nichterfindungsgemäßes Beispiel 1

Eine Lösung I wurde hergestellt, indem bei 80°C 2118,6 g Ammoniumheptamolybdat in 2,7 l Wasser gelöst wurde. Eine Lösung II wurde bei 80°C durch Lösen der gewünschten Menge an $H_2TeO_4 * 2 \ H_2O$ und Cobaltnitrat erhalten. Die beiden Lösungen wurden unter Rühren vereinigt und der sich bildende Slurry (Suspension) bis zur Trockene eingeengt. Das erhaltene noch feuchte Pulver wurde bei 150°C getrocknet und bei einer Temperatur von 600°C in die Oxide überführt. Das erhaltene Mischmetalloxidpulver besitzt die Zusammensetzung $(MoCo_{0,1}Te_{0,2})O_x$

Nichterfindungsgemäßes Beispiel 2

Der Katalysator des Beispiels 1 wurde mit einem Gemisch der Zusammensetzung von einem Anteil Propan (chemical grade) (50% der Gesamtmenge des Gemisches)und einem Anteil Sauerstoff (50% der Gesamtmenge des Gemisches) beaufschlagt. Die sich ergebende Raumgeschwindigkeit betrug 3000 h-1. Die Temperatur des Wärmeträgers betrug 450°C.Der Umsatz des Propans lag bei 48,9 mol-%, dabei betrug die Produktselektivität an Acrolein 32%.

Nichterfindungsgemäßes Beispiel 3

Eine Lösung I wurde hergestellt, indem bei 80°C 2118,6 g Ammoniumheptamolybdat in 2,7 l Wasser gelöst wurde. Eine Lösung II wurde bei 80°C durch Lösen der gewünschten Menge an Bi(III)-nitrat, und Chrom(III)-nitrat erhalten. Die beiden Lösungen wurden unter Rühren vereinigt und der sich bildende Slurry bis zur Trockene eingeengt. Das erhaltene noch feuchte Pulver wurde bei 150°C getrocknet und bei einer Temperatur von 600°C in die Oxide überführt. Das erhaltene Mischmetalloxidpulver besitzt die Zusammensetzung $(MoCr_{0,0286}Bi_{0,05})O_x$

Nichterfindungsgemäßes Beispiel 4

Der Katalysator des Beispiels 3 wurde mit einem Gemisch der Zusammensetzung von einem Anteil Propan (chemical grade) und einem Anteil Sauerstoff beaufschlagt. Die sich ergebende Raumgeschwindigkeit betrug 3000 h-1. Die Temperatur des Wärmeträgers betrug 500°C. Der Umsatz des Propans lag bei 38 mol-%, dabei betrug die Produktselektivität an Acrolein 43%.

Nichterfindungsgemäßes Beispiel 5

Das Produktgas des Beispiels 4 wurde recycliert und über den Katalysator des Beispiels 3 umgesetzt. Die sich ergebende Raumgeschwindigkeit betrug 3000 h-1. Die Temperatur des Wärmeträgers betrug 500°C. Der Umsatz des Propans lag bei 76 mol-%, dabei betrug die Produktausbeute an Acrolein 33 %.Beispiel 6

Eine Lösung I wurde hergestellt, indem bei 80°C 2118,6 g Ammoniumheptamolybdat in 2,7 l Wasser gelöst wurde. Eine Lösung II wurde bei 80°C durch Lösen der gewünschten Menge an Cr(III)-nitrat, und $H_2TeO_4$ * 2 $H_2O$ erhalten. Die beiden Lösungen wurden unter Rühren vereinigt und der sich bildende Slurry bis zur Trockene eingeengt. Das erhaltene noch feuchte Pulver wurde bei 150°C getrocknet und bei einer Temperatur von 600°C in die Oxide überführt. Das erhaltene Mischmetalloxidpulver besitzt die Zusammensetzung $(MoCr_{0,0286}Te_{0,05})O_x$

Nichterfindungsgemäßes Beispiel 7

Der Katalysator des Beispiels 6 wurde mit einem Gemisch der Zusammensetzung von einem Anteil Propan (chemical grade) und einem Anteil Sauerstoff beaufschlagt. Die sich ergebende Raumgeschwindigkeit betrug 3000 h-1. Die Temperatur des Wärmeträgers betrug 450°C. Der Umsatz des Propans lag bei 27,5 mol-%, dabei betrug die Produktselektivität an Acrolein 58%.

Erfindungsgemäßes Beispiel 8

Eine Lösung I wurde hergestellt, indem bei 80°C 2118,6 g Ammoniumheptamolybdat in 2,7 l Wasser gelöst wurde. Eine Lösung II wurde bei 80°C durch Lösen der gewünschten Menge an Cr(III)-nitrat, $H_3PO_4$ und $H_2TeO_4$ * 2 $H_2O$ erhalten. Die beiden Lösungen wurden unter Rühren vereinigt und der sich bildende Slurry bis zur Trockene eingeengt. Das erhaltene noch feuchte Pulver wurde bei 150°C getrocknet und bei einer Temperatur von 600°C in die Oxide überführt. Das erhaltene Mischmetalloxidpulver besitzt die Zusammensetzung $(MoCr_{0,0286}Te_{0,05}P_{0,05})O_x$

Erfindungsgemäßes Beispiel 9

Der Katalysator des Beispiels 8 wurde mit einem Gemisch der Zusammensetzung von einem Anteil Propan (chemical grade) und einem Anteil Sauerstoff beaufschlagt. Die sich ergebende Raumgeschwindigkeit betrug 3000 h-1. Die Temperatur des Wärmeträgers betrug 450°C. Der Umsatz des Propans lag bei 30 mol-%, dabei betrug die Produktselektivität an Acrolein 70%.

**Patentansprüche**

1. Verwendung von Mischoxidkatalysatoren zur katalytischen Gasphasenoxidation von Alkanen, **dadurch gekennzeichnet, dass** man einen Mischoxidkatalysator der allgemeinen Formel

$$(Mo_1Cr_{0,005-0,1} P_{0,01-0,1}Te_{0,01-0,8}G_g)O_x \qquad (I),$$

in der bedeuten:

G: mindestens eines der Elemente Si, Al, Ti, Zr
und

$$g = 0 - 800,$$

x = Zahl, die von der Wertigkeit und Häufigkeit der von Sauerstoff verschiedenen Elemente bestimmt wird,

verwendet.

**2.** Verwendung gemäß Anspruch 1, **dadurch gekennzeichnet, dass** man Propan zu Acrolein oder Acrolein und Acrylsäure oxidiert.

**3.** Verwendung gemäß Anspruch 1, **dadurch gekennzeichnet, dass** man Isobutan zu Methacrolein oder Methacrolein und Methacrylsäure oxidiert.

**4.** Verwendung gemäß den Ansprüchen 1 bis 3, **dadurch gekennzeichnet, dass** man den Katalysator mit einem Reaktionsgasgemisch beaufschlagt, das Alkan, Sauerstoff und Inertgas und Wasser in einem Verhältnis von 1:0,5-5:0-10:0-18 enthält.

**Claims**

**1.** Use of mixed oxide catalysts for catalytic gas phase oxidation of alkanes, **characterized in that** a mixed oxide catalyst of the general formula

$$(Mo_1Cr_{0.005-0.1}P_{0.01-0.1}Te_{0.01-0.8}G_g)O_x \qquad (I)$$

in which:

G: is at least one of the elements Si, Al, Ti, Zr
and

$$g = 0\text{-}800,$$

x = number which is determined by the valence and frequency of the elements other than oxygen,

is used.

**2.** Use according to Claim 1, **characterized in that** propane is oxidized to acrolein or acrolein and acrylic acid.

**3.** Use according to Claim 1, **characterized in that** isobutane is oxidized to methacrolein or methacrolein and methacrylic acid.

**4.** Use according to Claims 1 to 3, **characterized in that** the catalyst is contacted with a reaction gas mixture containing alkane, oxygen and inert gas and water in a ratio of 1:0.5-5:0-10:0-18.

**Revendications**

**1.** Utilisation de catalyseurs à base d'oxyde mixte pour l'oxydation catalytique en phase gazeuse d'alcanes, **caractérisée en ce qu'**un catalyseur à base d'oxyde mixte de formule générale

$$(Mo_1Cr_{0,005-0,1}P_{0,01-0,1}Te_{0,01-0,8}G_g)O_x \qquad (I)$$

dans laquelle :

G : signifie au moins un des éléments Si, Al, Ti, Zr
et

$$g = 0 \text{ à } 800,$$

x = un nombre déterminé par la valence et la fréquence des éléments différents de l'oxygène,

est utilisé.

**2.** Utilisation selon la revendication 1, **caractérisée en ce que** du propane est oxydé en acroléine ou en acroléine et acide acrylique.

**3.** Utilisation selon la revendication 1, **caractérisée en ce que** de l'isobutane est oxydé en méthacroléine ou en méthacroléine et acide méthacrylique.

**4.** Utilisation selon les revendications 1 à 3, **caractérisée en ce que** le catalyseur est chargé avec un mélange réactionnel gazeux qui contient un alcane, de l'oxygène et un gaz inerte et de l'eau en un rapport de 1:0,5-5:0-10:0-18.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 3776952 A
- FR 1447982 A
- GB 1086523 A
- GB 1286083 A
- WO 2007042369 A1
- EP 1075871 A1
- WO 2004105938 A2
- WO 2006058998 A2

- US 20050065370 A1
- US 20060004226 A1
- US 20030236163 A1
- WO 9920590 A1
- EP 0995491 A
- EP 1147807 A
- EP 1234612 A

**In der Beschreibung aufgeführte Nicht-Patentliteratur**

- *Xiamen Daxue Xuebao, Ziran Kexueban,* 2004, vol. 43 (2), 203-207
- *Cuihua Xuebao,* 2002, vol. 23 (3), 281-284

- **REDLINGSHOEFER H. ; FISCHER A. et al.** *Ind. Eng. Chem. Res.,* 2003, vol. 42, 5482-5488